# EUROPÄISCHE PATENTANMELDUNG

(11) **EP 1 223 532 A2**
(43) Veröffentlichungstag der Anmeldung: **17.07.2002**
(21) Anmeldenummer: 01129549.0
(22) Anmeldetag: 11.12.2001
(51) Int. Cl.: G06F 19/00

(54) **Verfahren und medizinisches System zur Versorgung eines Patienten mit einem Medikament**

(30) Priorität: 15.01.2001 DE 10101580
(71) Anmelder: SIEMENS AKTIENGESELLSCHAFT, 80333 München (DE)
(72) Erfinder: Redel, Thomas, Dr., 91099 Poxdorf (DE)

(57) **Zusammenfassung**

Die Erfindung betrifft ein Verfahren und ein medizinisches System zur Versorgung eines Patienten mit einem Medikament, aufweisend Mittel (5, 8, 9) zur Informationsübertragung für die Übertragung einer Menge und Dosierung des Medikaments (M1, M2) an eine Datenbank (7), welche Mittel zur Berechnung des Zeitpunktes, an dem das Medikament (M1, M2) aufgebraucht ist, aufweist, und Mittel (7, 12, 13, 14) zur Kontaktierung des Patienten (1) und/oder einer den Patienten (1) betreuende Person und/oder Einrichtung.

## Beschreibung

Die Erfindung betrifft ein Verfahren und ein medizinisches System zur Versorgung eines Patienten mit einem Medikament.

Bei einer medikamentösen Behandlung, insbesondere eines chronisch kranken Patienten, muss der Patient, wenn ein Vorrat eines für die Behandlung verwendeten Medikamentes zu Ende geht, einen den Patienten behandelnden Arzt aufsuchen, um ein neues Medikament verschrieben zu bekommen. Um diesen Arzttermin rechtzeitig zu vereinbaren, muss der Patient oder eine den Patienten betreuende Person oder Einrichtung den Vorrat des Medikamentes regelmäßig überwachen. Zur Unterstützung kann zwar ein elektronischer Gesundheitsassistent eingesetzt werden, welcher den voraussichtlichen Zeitpunkt berechnet, an dem das Medikament verbraucht sein wird, eine Koordination von Arztterminen, insbesondere wenn der Patient mehrere Medikamente einnehmen muss, bleibt dagegen mühsam und eine zuverlässige Versorgung des Patienten mit notwendigen Medikamenten relativ schwierig.

Eine automatische Medikamenten-Ausgabevorrichtung mit einer Mikroverarbeitungseinrichtung ist beispielsweise in der DE 690 17 365 T2 beschrieben. Die Medikamenten-Ausgabevorrichtung ist für eine Bereitstellung und Ausgabe von Medikamenten insbesondere in einem Krankenhaus vorgesehen. Sie kann derart ausgelegt sein, dass sie automatisch meldet, wenn die Menge bereitgestellter Medikamente einen vorbestimmten unteren Wert erreicht.

Aus der US 5,710,551 ist ein System bekannt, mit dessen Hilfe überprüft werden kann, ob ein Patient ein ihm verschriebenes Medikament wie verordnet einnimmt. Das System umfasst eine Ausgabevorrichtung, die eine Nachricht über eine Fernübertragungseinrichtung an eine zentrale Überwachungseinrichtung sendet, wenn der Patient sein verschriebenes Medikament der Ausgabevorrichtung entnimmt. Aufgrund der gesendeten und von der zentralen Überwachungseinrichtung empfangenen Nachrichten kann automatisch überprüft werden, ob der Patient sein Medikament wie verordnet einnimmt.

Ein weiteres System, mit dessen Hilfe überwacht werden kann, ob ein Patient das ihm verschrieben Medikament wie verordnet einnimmt, ist in der DE 693 11 658 T2 offenbart. Das System umfasst eine tragbare Datensammeleinheit und eine Daten verwertende Vorrichtung. Mit der tragbaren Datensammeleinheit werden Informationen im Zusammenhang mit einer Einnahme des verschriebenen Medikaments gesammelt und an die Daten verwertende Vorrichtung übermittelt. Die Daten verwertende Vorrichtung überwacht aufgrund der an sie übermittelten Informationen, ob der Patient das Medikament wie verordnet einnimmt.

In der WO 00/07538 ist ein Arzneimittelbehälter mit einer Codeleseeinrichtung offenbart. Bei einer Entnahme eines Medikamentes liest die Codeleseeinrichtung eine arzneispezifisch abgespeicherte Information aus. Zusammen mit dem Entnahmezeitpunkt dient diese Information ebenfalls für eine Überprüfung, ob der Patienten das ihm verschrieben Medikament wie verordnet einnimmt.

Des Weiteren ist eine Verwendung eines interaktiven Informationssystems zur Vorbereitung von Therapien im medizinischen und psychologischen Bereich in der DE 44 30 164 C1 offenbart. Das interaktive Informationssystem umfasst wenigstens einen Computer, wenigstens eine Schnittstelle zum Anschluss des Computers an ein privates oder öffentliches Telefonnetz, ein Computerprogramm zum Auswerten und umsetzen der über das Telefonnetz einspeisbaren Eingabesignale und wenigstens eine Ausgabevorrichtung, mit der in digitaler Form abgespeicherte Geräusche wiedergegeben werden können. Das interaktive Informationssystems fragt die Identität eines Patienten ab und protokolliert sie. Daraufhin fragt es personen- und diagnosespezifische Informationen ab und fertigt für den behandelnden Arzt oder Psychologen ein Therapieprotokoll an.

Ferner ist aus der JP 11120261 A ein System, mit dessen Hilfe ein Patient einen Arzt kontaktieren kann, bekannt. Wenn der Patient einen Arzt kontaktieren möchte, werden dem Patienten auf einem Terminal Namen verschiedener Ärzte, die der Patient kontaktieren kann, angeboten. Nachdem der Patient einen der Namen ausgewählt hat, wird das Terminal des Patienten automatisch mit einem Terminal des ausgewählten Arztes verbunden.

Die Aufgabe der Erfindung ist daher, ein Verfahren anzugeben und ein medizinisches System derart auszubilden, mit deren Hilfe Voraussetzungen geschaffen werden, eine zuverlässige Versorgung eines Patienten mit Medikamenten zu gewährleisten.

Nach der Erfindung wird die Aufgabe gelöst durch ein Verfahren zur Versorgung eines Patienten mit einem Medikament, aufweisend folgende Verfahrensschritte:
a) Übermitteln einer Angabe über eine Menge und eine Dosierung des Medikaments an eine Datenbank,
b) basierend auf der Angabe über die Menge und die Dosierung des Medikaments Berechnung des Zeitpunkts durch die Datenbank, an dem das Medikament aufgebraucht sein wird, und
c) basierend auf diesem Zeitpunkt Kontaktieren des Patienten und/oder eine den Patienten betreuende Person und/oder Einrichtung derart, dass dem Patienten ein neues Medikament rechtzeitig zur Verfügung gestellt werden kann.

Erfindungsgemäß werden also die Menge und Dosierung des Medikamentes zur Behandlung des Patienten einer Datenbank übermittelt. Unter Menge des Medikaments wird beispielsweise bei einem Medikament in Tablettenform die Anzahl der von dem behandelnden Arzt verschriebenen Tabletten oder bei einem flüssigen Medikament das Gesamtvolumen des unverbrauchten Medikaments verstanden. Die Dosierung des Medikamentes bezieht sich auf die Menge des Medikamentes, die der Patient zur Behandlung regelmäßig einnehmen muss, also beispielsweise die Anzahl der täglich einzunehmenden Tabletten. Die Datenbank kann sich beispielsweise in einer Arztpraxis des den Patienten behandelnden Arzt befinden und beispielsweise durch den Arzt, seine Assistenten oder einem Kundendienst gepflegt werden. Gemäß einer Ausführungsform kann die Datenbank aber auch von einem Service-Anbieter betrieben werden. Ein solcher Service-Anbieter kann in besonders günstiger Weise die Datenbank pflegen oder weitere Dienste anbieten. So ist es beispielsweise denkbar, dass der Service-Anbieter ein Call-Center betreibt, an das Fragen des Patienten, der den Patienten betreuenden Personen oder Einrichtung oder des Arztes gerichtet werden können. Folglich kann eine Betreuung des Patienten effektiver gestaltet werden und bei Fragen schnell, freundlich und zuverlässig weitergeholfen werden. Die Menge und Dosierung des Medikaments kann dem Service-Anbieter beispielsweise durch den Patienten, eine den Patienten betreuende Person oder Einrichtung oder durch den Arzt oder einen seiner Assistenten beispielsweise telefonisch oder per e-mail übermittelt werden. Gemäß einer vorteilhaften Ausführung der Erfindung werden diese Informationen auch automatisch von der Arztpraxis zur Datenbank übermittelt. So wäre es denkbar, dass ein Rechner der Arztpraxis, mit dem ein Rezept für das Medikament ausgestellt wird, automatisch die Datenbank, beispielsweise über Internet, kontaktiert und die Menge und Dosierung des Medikamentes sowie die Identität des Patienten übermittelt. Somit ist insbesondere sichergestellt, dass die Datenbank stets aktualisiert ist. Anschließend berechnet die Datenbank, basierend auf der Dosierung und der Menge des Medikamentes, den Zeitpunkt, an dem das Medikament aufgebraucht sein wird. Die Datenbank kann ebenfalls eine eventuelle Restmenge eines noch unverbrauchten früher verschriebenen Medikamentes berücksichtigen. Basierend auf dem errechneten Zeitpunkt kann dann der Arzt oder einer seiner Assistenten oder, wenn die Datenbank von dem Service-Anbieter betrieben wird, ein Mitarbeiter des Service-Anbieters den Patienten und/oder die den Patienten betreuende Person und/oder Einrichtung kurz vor diesem Zeitpunkt z.B. telefonisch oder per e-mail kontaktieren, um auf den bevorstehenden Verbrauch des Medikamentes hinzuweisen, so dass beispielsweise der Patient einen Arzttermin vereinbaren kann. Somit muss der Patient oder die den Patienten betreuende Person oder Einrichtung den Vorrat des Medikamentes nicht selber überwachen. Folglich sind Vorraussetzungen geschaffen, dass dem Patienten das Medikament nicht versehentlich ausgeht und somit seine Behandlung beeinträchtigt wird.

Bei einer Variante der Erfindung ist vorgesehen, dass basierend auf dem Zeitpunkt des Aufbrauchens des Medikaments ein Arzttermin bei dem Arzt für den Patienten vereinbart wird. Der Arzttermin wird in vorteilhafter Weise kurz vor dem Zeitpunkt gelegt, an dem das Medikament aufgebraucht ist. Der Arzttermin kann beispielsweise als Service-Leistung durch den Mitarbeiter des Service-Anbieters oder von dem Arzt oder seinen Assistenten vereinbart werden.

Gemäß einer besonders praktischen Variante der Erfindung vereinbart die Datenbank automatisch den Arzttermin. Somit können beispielsweise Mitarbeiter des Service-Anbieters eingespart werden.

Nach einer weitern Ausführungsform der Erfindung kann die Datenbank einen elektronischen Terminkalender des Arztes kontaktieren und basierend auf den in dem elektronischen Terminkalender gespeicherten Informationen den Arzttermin vereinbaren. Somit ist sicher gestellt, dass bei der automatisierten Vereinbarung des Arzttermins der Arzt verfügbar ist. Ein weiterer Vorteil der automatisierten Vereinbarung des Arzttermins ist, dass sich keine weitere Person oder der Patient selbst um die Vereinbarung des Arzttermins kümmern muss. Folglich kann eine Vereinbarung des Arzttermins auch nicht versehentlich vergessen werden. Außerdem können durch das Kontaktieren des Terminkalenders insbesondere längere Abwesenheitszeiten des Arztes, beispielsweise wegen Urlaub, bei der Planung des Arzttermins frühzeitig berücksichtigt werden, so dass beispielsweise ein den behandelnden Arzt vertretender Arzt aufgesucht werden kann. Folglich sind Vorraussetzungen geschaffen, den Arzttermin auf einen Zeitpunkt zu legen, so dass der Patient zuverlässig mit einem neuen Medikament versorgt wird, bevor das Medikament verbraucht ist.

Bei einer weiteren günstigen Variante der Erfindung ist außerdem vorgesehen, dass die Datenbank denjenigen Zeitraum für den Arzttermin berücksichtigt, an dem der Patient verhindert ist. Somit ist auch die Verfügbarkeit des Patienten sicher gestellt und es kann insbesondere vermieden werden, dass der Arzttermin auf einen Zeitraum gelegt wird, an dem der Patient beispielsweise selber verreist ist.

Bei einer weiteren Variante der Erfindung ist außerdem vorgesehen, dass die Datenbank den Patienten und/oder eine den Patienten betreuende Person und/oder Einrichtung automatisch von dem Arzttermin informiert. Zu diesem Zwecke ist es denkbar, dass der Patient oder die den Patienten betreuende Person oder Einrichtung beispielsweise automatisch mittels e-mail von dem Arzttermin informiert wird.

Eine weitere Variante der Erfindung sieht vor, dass der Arzttermin bei der Datenbank bestätigt werden muss. Somit ist insbesondere sichergestellt, dass sowohl der Patient als auch der Arzt den Zeitpunkt des Arzttermins zuverlässig erhalten oder dass der Arzttermin aus wichtigen Gründen verschoben werden kann. So ist es beispielsweise denkbar, dass der Patient eine geringere Menge des Medikaments einnahm, beispielsweise bedingt durch einen längeren Krankenhausaufenthalt, und die Datenbank darüber nicht informiert wurde, weil dies beispielsweise der Patient vergaß oder das Krankenhaus nicht mit der Datenbank verbunden ist. Folglich verfügt der Patient noch über eine größere Restmenge des Medikaments als die Datenbank berechnete und der Arzttermin kann zu einem späteren Zeitpunkt erfolgen.

Bei einer weiteren Ausführungsform der Erfindung ist vorgesehen, dass der Arzt automatisch informiert wird, wenn der Patient nicht rechtzeitig das neue Medikament zur Verfügung gestellt bekommt. Folglich kann der Arzt beispielsweise den Patienten direkt kontaktieren, um die Behandlung des Patienten nicht zu gefährden.

Bei einer besonders günstigen Variante der Erfindung ist vorgesehen, dass, wenn der Patient wenigstens ein weiteres Medikament einnimmt und/oder medizinische Utensilien benützt, der Datenbank eine Menge und Dosierung des weiteren Medikaments oder der weiteren Medikamente und/oder eine Menge und ein Verbrauch der medizinischen Utensilien mitgeteilt wird, die Datenbank basierend auf der Angabe der Menge und Dosierung des weiteren Medikaments oder der weiteren Medikamente und/oder basierend auf der Angabe der Menge und des Verbrauchs der medizinischen Utensilien den Zeitpunkt oder die Zeitpunkte berechnet, an dem das weitere Medikament oder an denen die weiteren Medikamente und/oder die medizinischen Utensilien verbraucht ist oder sind, und basierend auf den Zeitpunkten einen Arzttermin berechnet, so dass der Arzt dem Patienten rechtzeitig ein oder mehrere neue Medikamente und/oder neue medizinische Utensilien verschreiben kann und die Anzahl der Arzttermine minimiert wird. Folglich werden häufige, zeitlich nahe zusammenliegende Arzttermine vermieden, wodurch dem Patienten und/oder dem Arzt kostbare Zeit gespart und somit Behandlungskosten reduziert werden. Unter medizinische Utensilien werden im Übrigen beispielsweise Spritzen, wie sie z.B. Diabetiker benötigen, oder Tupfer verstanden.

Die Aufgabe der Erfindung wird auch gelöst durch ein medizinisches System zur Versorgung eines Patienten mit einem Medikament, aufweisend Mittel zur Informationsübertragung für die Übertragung einer Angabe über eine Menge und eine Dosierung des Medikaments an eine Datenbank, welche Mittel zur Berechnung des Zeitpunktes, an dem das Medikament aufgebraucht sein wird, aufweist, und Mittel zur Kontaktierung des Patienten und/oder einer den Patienten betreuende Person und/oder Einrichtung. Die Mittel zur Informationsübertragung können beispielsweise das Internet oder ein Telefon, mit dem die Menge und Dosierung des Medikaments einer die Datenbank betreibenden Person oder Einrichtung fernmündlich übermittelt werden, umfassen. Die Datenbank berechnet basierend auf diesen Informationen den Zeitpunkt, an dem das Medikament verbraucht ist. Des weiteren weist das medizinische System Mittel zur Kontaktierung des Patienten und/oder einer den Patienten betreuende Person und/oder Einrichtung auf. Die Mittel zur Kontaktierung können beispielsweise das Internet, ein Faxgerät, ein Telefon, o.ä. umfassen. Mit den Mitteln zur Kontaktierung können der Patienten und/oder eine den Patienten betreuende Person und/oder Einrichtung insbesondere rechtzeitig verständigt werden, so dass dem Patienten ein neues Medikament rechtzeitig zur Verfügung gestellt werden kann.

Weitere Vorteile der Erfindung ergeben sich aus den Unteransprüchen.

Ein Ausführungsbeispiel der Erfindung ist in der beigefügten schematischen Zeichnung dargestellt.

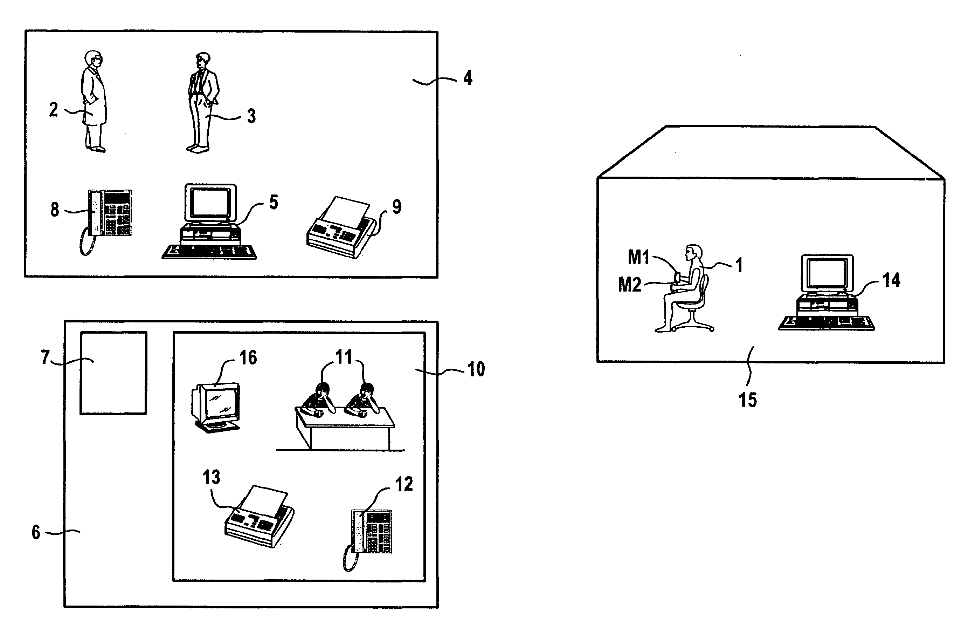

Die Figur zeigt ein Schaubild eines erfindungsgemäßen medizinisches Systems, anhand dessen auch das erfindungsgemäße Verfahren erläutert wird. Einem in der Figur schematisch dargestellten Patienten 1 wurde von seinem Arzt 2 ein Medikament M1 und ein Medikament M2 verschrieben. Ein für die Medikamente M1 und M2 notwendiges und in der Figur nicht dargestelltes Rezept wurde von dem Arzt 2 oder einer den Arzt 2 unterstützenden Person 3 mit Hilfe eines in einer Arztpraxis 4 des Arztes 2 angeordneten Rechners 5 ausgestellt. Das Rezept umfasste neben Angaben zur Identität der Person 1 auch die Bezeichnungen, die Mengen und die verschriebenen Dosierungen der Medikamente M1 und M2.

Im Falle des vorliegenden Ausführungsbeispiels kontaktierte der Rechner 5 nach Erstellen des Rezeptes automatisch eine von einem Service-Anbieter 6 betriebene Datenbank 7 über eine in der Figur nicht dargestellte Internetverbindung und übermittelte der Datenbank 7 die Art, Mengen und verschrieben Dosierungen der Medikamente M1 und M2 sowie die Identität des Patienten 1. Insbesondere bei einer längeren Störung der Internetverbindung können der Arzt 2 oder die Person 3 diese Angaben auch mit einem der Arztpraxis 4 zugeordneten Telefon 8 und/oder Faxgerät 9 fernmündlich bzw. schriftlich einem dem Service-Anbieter 6 zugeordneten Call-Center 10 übermitteln. In dem Call-Center 10 arbeiten Personen 11, die Zugang zu einem in dem Call-Center 10 angeordneten Telefon 12 und Faxgerät 13 haben. Aufgrund dieser Angaben errechnete die Datenbank 7 automatisch die Zeitpunkte, an denen die Medikamente M1 und M2 verbraucht sein werden. Zur Berechnung dieser Zeitpunkte berücksichtigte die Datenbank 7 ebenfalls eventuelle Restmengen von Medikamenten der gleichen Sorte wie die Medikamente M1 und/oder M2, die der Patient 1 an dem Zeitpunkt noch übrig hatte, an dem ihm die Medikamente M1 und M2 verschrieben wurden. Insbesondere wenn der Patient 1 noch unvorhergesehen größere Restmengen von Medikamenten übrig hatte, z.B. bedingt durch einen längeren Krankenhausaufenthalt, bei dem der Patient 1 von dem Arzt 2 verschriebene Medikamente nicht einnahm, wurden die Angaben zur Restmenge der Medikamente von dem Arzt 2, der Person 3 oder dem Patienten 1 dem Call-Center 10 mittels e-mail, telefonisch oder per Fax mitgeteilt, so dass einer der Personen 11 die Datenbank 7 entsprechend aktualisieren konnte.

Basierend auf den Zeitpunkten plante die Datenbank 7 einen erneuten Arzttermin für den Patienten 1 derart, dass dem Patienten 1 in der Figur nicht dargestellte neue Medikamente rechtzeitig verschrieben werden können, bevor die Medikamente M1 oder M2 verbraucht sind. Die neuen Medikamente sind von der gleichen Sorte wie die Medikamente M1 und M2. Für die Planung des Arzttermins fragte im Falle des vorliegenden Ausführungsbeispiels die Datenbank 7 automatisch einen auf dem Rechner 5 gespeicherten elektronischen Terminkalender des Arztes 2 ab, um die Verfügbarkeit des Arztes 2 sicher zu stellen. Um sich ferner der Verfügbarkeit des Patienten 1 zu vergewissern, fragte im Falle des vorliegenden Ausführungsbeispiels die Datenbank 7 ebenfalls einen auf einem mit dem Internet verbundenen Rechner 14 gespeicherten elektronischen Terminkalender des Patienten 1 ab. Der Rechner 14 ist im Falle des vorliegenden Ausführungsbeispiels in einem Haus 15 angeordnet, welches der Patient 1 bewohnt.

Nachdem der Arzttermin, welcher vor dem Zeitpunkt liegt, an dem eines der Medikamente M1 oder M2 ausgeht, geplant ist, wurde im Falle des vorliegenden Ausführungsbeispiels der Arzttermin dem Patienten 1 automatisch von der Datenbank 7 per e-mail mitgeteilt und beim Arzt 2 per e-mail vorgemerkt. Bevor der Arzttermin bei dem Arzt 2 bestätigt werden kann, muss der Patient 1 den Arzttermin mittels e-mail bei der Datenbank 7 bestätigen, damit die Datenbank 7 automatisch den Arzttermin auf dem elektronischen Terminkalender des Arztes 2 bestätigen kann. Sollte der Patient 1 den Arzttermin nicht bestätigen, ist im Falle des vorliegenden Ausführungsbeispiels vorgesehen, dass eine der Personen 11 des Call-Centers 10 den Patienten 1 telefonisch ca. 2 Wochen vor dem Arzttermin an den Arzttermin erinnert. Sollte der Patient 1 den Arzttermin trotzdem nicht bestätigen, ist im Falle des vorliegenden Ausführungsbeispiels ferner vorgesehen, dass beim Verstreichen des Arzttermins die Datenbank 7 automatisch dem Arzt 2 eine e-mail von der fehlenden Bestätigung des Patienten 2 sendet. Somit ist insbesondere sicher gestellt, dass der Arzt 2 von einem baldigen Verbrauch des Medikaments M1 und/oder M2 des Patienten 1 informiert ist und kann sich, wenn er es für nötig hält, direkt mit dem Patienten 1 in Verbindung setzen.

Wenn der Patient 1 den Arzttermin wahrnimmt und neue Medikamente vom Arzt 2 verschrieben bekommt, wird, wie bereits obenstehend beschrieben, erneut die Datenbank 7 derart kontaktiert, dass die Mengen und Dosierungen der neuen Medikamente der Datenbank 7 mitgeteilt werden, so dass die Datenbank 7 einen erneuten Arzttermin berechnen kann.

Abweichend von dem obenstehend beschriebenen Verfahren ist es auch möglich, dass die in dem Call-Center 10 arbeitenden Personen 11 die Zeitpunkte überwachen, an denen Medikamente von Kunden des Service-Anbieters 6, also u.a. des Patienten 1, verbraucht sind. Beispielsweise vier Wochen bevor eines der Medikamente M1 oder M2 des Patienten 1 verbraucht ist, erhält das Call-Center 10 einen entsprechenden Hinweis auf einem mit der Datenbank 7 in der Figur in nicht dargestellter Weise verbundenen Monitor 16. Einer der Personen 11 des Call-Centers 10 informiert daraufhin den Patienten 1 von dem bevorstehenden Verbrauch einer der Medikamente M1 oder M2. Insbesondere wenn der Patient 1 telefonisch benachrichtigt wird, kann, da die Datenbank 7 auf den elektronischen Terminkalender des Arztes 2 zugreifen kann, einer der Personen 11 des Call-Centers 10 den Arzttermin für den Patienten 1 im Wesentlichen sofort als Serviceleistung vereinbaren. Des weiteren kann eine der Personen 11 den Patienten 1 mit für die Einnahme der Medikamente M1 und M2 relevante Informationen, wie Verträglichkeit, Nebenwirkungen, usw. versorgen.

Wenn die Datenbank 7 nicht auf den elektronischen Terminkalender des Arztes 2 zugreifen kann, ist es auch möglich, dass einer der Personen 11 den Arzttermin konventionell vereinbart, indem sie den Arzt 2 oder die Person 3 mittels des Telefons 12 anruft. Der Arzttermin kann auch von dem Patienten 1 selber vereinbart werden.

Es ist aber auch denkbar, dass der Patient 1 per Internet Zugriff auf die Datenbank 7 hat und auf eine von dem Service-Anbieter 6 gepflegten und dem Patienten 1 zugeordnete Internetseite zugreifen kann, auf der beispielsweise neben dem Zeitpunk, an dem wenigstens eines der Medikamente M1 oder M2 verbraucht sein wird, auch andere Informationen zu finden sind, wie beispielsweise Beschreibungen, Dosierungen, Wirkungsweisen, Nebenwirkungen, usw. der Medikamente M1 und M2.

Es ist aber auch möglich, dass der Patient 1 regelmäßig das Call-Center 10 telefonisch kontaktiert, wobei einer der Personen 11 den Patienten 1 rechtzeitig von dem Verbrauch der Medikamente M1 oder M2 informiert.

Es ist aber auch denkbar, dass das Haus 15 ein Pflege- oder Altenheim ist, in dem der Patient 1 untergebracht ist. Dann ist es auch denkbar, dass beispielsweise das Call-Center 10 und/oder die Datenbank 7 mit einer den Patienten 1 betreuenden Person oder die Person 1 betreuende Einrichtung wie z.B. das Pflege- oder Altenheim, in Kontakt steht.

Die Menge und Dosierung der Medikamente M1 und M2 können auch von dem Patienten 1, einer den Patienten 1 betreuenden Person und/oder Einrichtung übermittelt werden.

Auch ist ein automatisches Informieren des Arztes 2 durch die Datenbank 7 von einem nicht bestätigten Arzttermin optional. Es ist auch möglich, dass das Call-Center 10 den Arzt 2 informiert.

Die Datenbank 7 muss auch nicht notwendigerweise von einem Service-Anbieter 6 sondern kann auch insbesondere von dem Arzt 2 betrieben werden.

Der Service-Anbieter 6 muss auch nicht notwendigerweise ein Call-Center 10 betreiben.

Ein automatisches Kontaktieren der Datenbank 7 durch den Rechner 5 ist ebenfalls optional. Es ist auch möglich, dass der Arzt 2 oder die Person 3 die zur Berechnung relevanten Daten auf einer Internet-Homepage des Service-Anbieters 6 einträgt.

Das Verfahren bzw. das medizinische System ist auch für Tiere anwendbar.

Es können auch anstelle oder zusätzlich zu dem Medikament M1 und/oder M2 medizinische Utensilien, wie beispielsweise Spritzen oder Tupfer berücksichtigt werden.

Im Übrigen sind Insbesondere die Anzahl von zwei Medikamenten M1 und M2 und das obenstehende Ausführungsbeispiel exemplarisch zu verstehen.

## Patentansprüche

1. Verfahren zur Versorgung eines Patienten mit einem Medikament, aufweisend folgende Verfahrensschritte:
a) Übermitteln einer Angabe über eine Menge und eine Dosierung des Medikaments (M1, M2) an eine Datenbank (7),
b) basierend auf der Angabe über die Menge und die Dosierung des Medikaments (M1, M2) Berechnung des Zeitpunkts durch die Datenbank (7), an dem das Medikament (M1, M2) aufgebraucht sein wird, und
c) basierend auf diesem Zeitpunkt Kontaktieren des Patienten (1) und/oder eine den Patienten (1) betreuende Person und/oder Einrichtung derart, dass dem Patienten (1) ein neues Medikament rechtzeitig zur Verfügung gestellt werden kann.

2. Verfahren zur Versorgung eines Patienten mit einem Medikament nach Anspruch 1, bei welchem die Datenbank (7) von einem Service-Anbieter (6) betrieben wird.

3. Verfahren zur Versorgung eines Patienten mit einem Medikament nach Anspruch 1 oder 2, bei welchem die Datenbank (7) automatisch von einer Arztpraxis (4) des das Medikament (M1, M2) verschreibenden Arztes (2) kontaktiert wird.

4. Verfahren zur Versorgung eines Patienten mit einem Medikament nach einem der Ansprüche 1 bis 3, bei welchem basierend auf dem Zeitpunkt des Aufbrauchens des Medikaments (M1, M2) ein Arzttermin vereinbart wird.

5. Verfahren zur Versorgung eines Patienten mit einem Medikament nach einem der Ansprüche 1 bis 3, bei welchem die Datenbank (7) automatisch einen Arzttermin vereinbart.

6. Verfahren zur Versorgung eines Patienten mit einem Medikament nach einem der Ansprüche 1 bis 5, bei welchem die Datenbank (7) einen elektronischen Terminkalender des Arztes (2) kontaktieren kann und basierend auf den in dem elektronischen Terminkalender gespeicherten Informationen der Arzttermin vereinbart wird.

7. Verfahren zur Versorgung eines Patienten mit einem Medikament nach einem der Ansprüche 1 bis 6, bei welchem die Datenbank (7) denjenigen Zeitraum für den Arzttermin berücksichtigt, an dem der Patient (1) verhindert ist.

8. Verfahren zur Versorgung eines Patienten mit einem Medikament nach einem der Ansprüche 1 bis 7, bei welchem die Datenbank (7) den Patienten (1) und/oder eine den Patienten (1) betreuende Person und/oder Einrichtung automatisch von dem Arzttermin informiert.

9. Verfahren zur Versorgung eines Patienten mit einem Medikament nach einem der Ansprüche 1 bis 8, bei welchem der Arzttermin bei der Datenbank (7) bestätigt werden muss.

10. Verfahren zur Versorgung eines Patienten mit einem Medikament nach einem der Ansprüche 1 bis 9, bei welchem der Arzt (2) automatisch informiert wird, wenn der Patient (1) nicht rechtzeitig das neue Medikament zur Verfügung gestellt bekommt.

11. Verfahren zur Versorgung eines Patienten mit einem Medikament nach einem der Ansprüche 4 bis 10, bei welchem der Patient (1) wenigstens ein weiteres Medikament (M1, M2) einnimmt und/oder medizinische Utensilien benützt, der Datenbank (7) eine Menge und Dosierung des weiteren Medikaments (M1, M2) oder der weiteren Medikamente (M1, M2) und/oder eine Menge und ein Verbrauch der medizinischen Utensilien mitgeteilt wird, die Datenbank (7) basierend auf der Angabe der Menge und Dosierung des weiteren Medikaments (M1, M2) oder der weiteren Medikamente (M1, M2) und/oder basierend auf der Angabe der Menge und des Verbrauchs der medizinischen Utensilien den Zeitpunkt oder die Zeitpunkte berechnet, an dem das weitere Medikament (M1, M2) oder an denen die weiteren Medikamente (M1, M2) und/oder die medizinischen Utensilien verbraucht ist oder sind, und basierend auf den Zeitpunkten einen Arzttermin berechnet, so dass der Arzt (2) dem Patienten (1) rechtzeitig ein oder mehrere neue Medikamente und/oder neue medizinische Utensilien verschreiben kann und die Anzahl der Arzttermine minimiert wird.

12. Medizinisches System zur Versorgung eines Patienten mit einem Medikament, aufweisend
- Mittel (5, 8, 9) zur Informationsübertragung für die Übertragung einer Angabe über eine Menge und eine Dosierung des Medikaments (M1, M2) an eine Datenbank (7), welche Mittel zur Berechnung des Zeitpunktes, an dem das Medikament (M1, M2) aufgebraucht sein wird, aufweist, und
- Mittel (7, 12, 13, 14) zur Kontaktierung des Patienten (1) und/oder einer den Patienten (1) betreuende Person und/oder Einrichtung.

13. Medizinisches System zur Versorgung eines Patienten mit einem Medikament nach Anspruch 12, bei welchem die Datenbank (7) von einem Service-Anbieter (6) betrieben wird.

14. Medizinisches System zur Versorgung eines Patienten mit einem Medikament nach Anspruch 12 oder 13, bei welchem die Datenbank (7) automatisch mit einer Arztpraxis (4) eines das Medikament (M1, M2) verschreibenden Arztes (2) zugeordnete Mitteln (5, 8, 9) zur Informationsübertragung kontaktierbar ist.

15. Medizinisches System zur Versorgung eines Patienten mit einem Medikament nach einem der Ansprüche 12 bis 14, bei welchem basierend auf dem Zeitpunk des Aufbrauchens des Medikaments (M1, M2) ein Arzttermin vereinbart wird.

16. Medizinisches System zur Versorgung eines Patienten mit einem Medikament nach einem der Ansprüche 12 bis 14, bei welchem die Datenbank (7) automatisch einen Arzttermin vereinbart.

17. Medizinisches System zur Versorgung eines Patienten mit einem Medikament nach einem der Ansprüche 12 bis 16, bei welchem die Datenbank (7) einen elektronischen Terminkalender des Arztes (2) kontaktieren kann und basierend auf den in dem elektronischen Terminkalender gespeicherten Informationen der Arzttermin vereinbart wird.

18. Medizinisches System zur Versorgung eines Patienten mit einem Medikament nach einem der Ansprüche 12 bis 17, bei welchem die Datenbank (7) denjenigen Zeitraum für den Arzttermin berücksichtigt, an dem der Patient (1) verhindert ist.

19. Medizinischen System zur Versorgung eines Patienten mit einem Medikament nach einem der Ansprüche 12 bis 18, bei welchem der Patienten (1) und/oder eine den Patienten (1) betreuende Person und/oder Einrichtung automatisch mit den Mitteln (7, 12, 13, 14) zur Kontaktierung von der Datenbank (7) über den Arzttermin informierbar ist.

20. Medizinisches System zur Versorgung eines Patienten mit einem Medikament nach einem der Ansprüche 12 bis 19, bei welchem der Arzttermin bei der Datenbank (7) bestätigt werden muss.

21. Medizinisches System zur Versorgung eines Patienten mit einem Medikament nach einem der Ansprüche 12 bis 20, bei welchem der Arzt (2) mit dem Arzt (2) zugeordnete Mitteln (5, 8, 9) zur Informationsübertragung automatisch informierbar ist, wenn der Patient (1) nicht rechtzeitig das neue Medikament zur Verfügung gestellt bekommt.
